# EUROPEAN PATENT APPLICATION

(11) **EP 4 446 417 A1**
(43) Date of publication of application: **16.10.2024**
(21) Application number: 22904380.7
(22) Date of filing: 29.07.2022
(51) Int. Cl.: C12N 15/77, C12N 9/02, C12P 13/08

(54) **CORYNEBACTERIUM GLUTAMICUM VARIANT HAVING IMPROVED L-LYSINE PRODUCTION ABILITY AND METHOD FOR PRODUCING L-LYSINE BY USING SAME**

(30) Priority: 06.12.2021 KR 20210173249
(71) Applicant: Daesang Corporation, Seoul 02586 (KR)
(72) Inventor: KIM, Ha Eun, Icheon-si, Gyeonggi-do 17384 (KR); LEE, Sun Hee, Yongin-si, Gyeonggi-do 16923 (KR); LEE, Young Ju, Seoul 06250 (KR); KIM, Bong Ki, Seoul 07900 (KR); PARK, Seok Hyun, Namyangju-si, Gyeonggi-do 12258 (KR); PARK, Joon Hyun, Seongnam-si, Gyeonggi-do 13523 (KR)
(74) Representative: Cabinet Nony
(86) International application number: PCT/KR2022/011180
(87) International publication number: WO 2023/106543

(57) **Abstract**

The present invention relates to a *Corynebacterium glutamicum* mutant strain having enhanced L-lysine productivity and a method of producing L-lysine using the same. The *Corynebacterium glutamicum* mutant strain is able to produce L-lysine in an improved yield as a result of improving the activity of glyceraldehyde 3-phosphate dehydrogenase by mutagenesis of amino acids in the gene encoding glyceraldehyde 3-phosphate dehydrogenase.

## Description

### Technical Field

The present invention relates to a *Corynebacterium glutamicum* mutant strain having enhanced L-lysine productivity and a method of producing L-lysine using the same.

### Background Art

L-lysine is an essential amino acid that is not synthesized in the human or animal body. L-lysine needs to be supplied externally and is generally produced by fermentation using microorganisms such as bacteria or yeast. L-lysine production may be performed using naturally occurring wild-type strains or mutant strains obtained by modifying the wild-type strains to have enhanced L-lysine productivity. In recent years, in order to improve the production efficiency of L-lysine, various recombinant strains or mutant strains having excellent L-lysine productivity and methods of producing L-lysine using the recombinant strains or mutant strains have been developed by applying gene recombination technology to microorganisms such as *Escherichia coli* and *Corynebacterium,* which are widely used for the production of L-amino acids and other useful substances.

According to Korean Patent Nos. 10-0838038 and 10-2139806, L-lysine productivity may be enhanced by increasing the expression of genes encoding proteins, including L-lysine production-related enzymes, through modification of the nucleotide sequences of the genes or the amino acid sequences of the proteins, or by removing unnecessary genes. In addition, Korean Patent Application Publication No. 10-2020-0026881 discloses a method by which the existing promoter of a gene encoding an enzyme involved in L-lysine production is changed to a promoter having strong activity in order to increase the expression of the gene.

As described above, various methods for increasing L-lysine productivity have been developed, but there are dozens of types of proteins such as enzymes, transcription factors, and transport proteins, which are directly or indirectly involved in L-lysine production. Hence, extensive studies still need to be conducted on whether or not changes in the activities of these proteins lead to an increase in L-lysine productivity.

### [Prior Art Documents]

### [Patent Documents]

Korean Patent No. 10-0838038
Korean Patent No. 10-2139806
Korean Patent Application Publication No. 10-2020-0026881

### DISCLOSURE

### Technical Problem

An object of the present invention is to provide a *Corynebacterium glutamicum* mutant strain having enhanced L-lysine productivity.

Another object of the present invention is to provide a method of producing L-lysine using the mutant strain.

### Technical Solution

The present inventors have conducted studies to develop a novel mutant strain having enhanced L-lysine productivity using a *Corynebacterium glutamicum* strain, and as a result, have found that, when an amino acid at a specific position in the amino acid sequence of the gapA gene encoding glyceraldehyde 3-phosphate dehydrogenase involved in the L-lysine biosynthesis pathway is substituted, the production of L-lysine further increases, thereby completing the present invention.

One aspect of the present invention provides a *Corynebacterium glutamicum* mutant strain having enhanced L-lysine productivity by having improved activity of glyceraldehyde 3-phosphate dehydrogenase.

"Glyceraldehyde 3-phosphate dehydrogenase (GAPDH)" as used in the present invention refers to an enzyme that is involved in glycolysis or glycogenolysis during energy metabolism and catalyzes the reversible reaction of glyceraldehyde 3-phosphate to 1,3-bisphosphoglycerate and at the same time, reduces the coenzyme NAD+ or NADP+ to NADH or NADPH or, conversely, oxidizes NADH or NADPH. GAPDH is classified into three subtypes, GapA, GapB, and GapN, depending on the type of coenzyme. GapA catalyzes the reaction that not only produces 1,3-bisphosphoglycerate and NADH from glyceraldehyde 3-phosphate using NAD in glycolysis, but also produces glyceraldehyde 3-phosphate from 1,3-bisphosphoglycerate using NADH in gluconeogenesis, and GapB is activated by both NAD and NADP and functions only in gluconeogenesis. GapN is NADP-dependent and catalyzes the irreversible oxidation of glyceraldehyde 3-phosphate to 1,3-bisphosphoglycerate without producing ATP.

In the present invention, in order to produce or supply a large amount of NADPH required for the lysine biosynthesis pathway, a mutant strain, which has improved activity of GapA and produces NADPH using NADP instead of NAD in the conversion of glyceraldehyde 3-phosphate, was constructed.

According to one embodiment of the present invention, the glyceraldehyde 3-phosphate dehydrogenase may be GapA.

According to one embodiment of the present invention, the glyceraldehyde 3-phosphate dehydrogenase may be derived from a *Corynebacterium* sp. strain. Specifically, the *Corynebacterium* sp. strain may be, but is not limited to, *Corynebacterium glutamicum, Corynebacterium crudilactis, Corynebacterium deserti, Corynebacterium callunae, Corynebacterium suranareeae, Corynebacterium lubricantis, Corynebacterium doosanense, Corynebacterium efficiens, Corynebacterium uterequi, Corynebacterium stationis, Corynebacterium pacaense, Corynebacterium singulare, Corynebacterium humireducens, Corynebacterium marinum, Corynebacterium halotolerans, Corynebacterium spheniscorum, Corynebacterium freiburgense, Corynebacterium striatum, Corynebacterium canis, Corynebacterium ammoniagenes, Corynebacterium renale, Corynebacterium pollutisoli, Corynebacterium imitans, Corynebacterium caspium, Corynebacterium testudinoris, Corynebacaterium pseudopelargi,* or *Corynebacterium flavescens.*

"Improved activity" as used in the present invention means that a protein of interest such as an enzyme, transcription factor or transport protein is structurally modified so that the mutant strain produces a product or protein complex different from that produced by the wild-type strain or the strain before modification, or the concentration of product or protein complex increases compared to that in the wild-type strain or the strain before modification. Here, "structurally modified" means that the reaction site of a protein for a substrate or binding protein, for example, the active site of an enzyme is physically modified. This improved activity also includes: a case in which the activity of the protein itself is changed or increased compared to the activity of the protein of the parent microorganism by substitution, insertion, deletion, or a combination thereof of one or more nucleotides in the gene encoding the protein; a case in which the overall enzyme activity in the cell is higher than that in the wild-type strain or the strain before modification due to increased expression or translation of the gene encoding the protein; and a combination thereof.

According to one embodiment of the present invention, the improved activity of the glyceraldehyde 3-phosphate dehydrogenase may be achieved by site-directed mutagenesis of the gene encoding the glyceraldehyde 3-phosphate dehydrogenase.

According to one embodiment of the present invention, the gene encoding the glyceraldehyde 3-phosphate dehydrogenase may be represented by the amino acid sequence of SEQ ID NO: 1.

According to one embodiment of the present invention, the gene encoding the glyceraldehyde 3-phosphate dehydrogenase may be represented by the nucleotide sequence of SEQ ID NO: 2.

According to one embodiment of the present invention, the improved activity of the glyceraldehyde 3-phosphate dehydrogenase may be achieved by substitution of one or more amino acids in the 10^{th} to 130^{th} amino acid region in the amino acid sequence of the gene encoding the glyceraldehyde 3-phosphate dehydrogenase.

More specifically, the genetic mutation in the present invention may be achieved by substitution of one or more amino acids in the 10^{th} to 130^{th} amino acid region, preferably substitution of 1, 2, 3, 4, or 5 consecutive or non-consecutive amino acids in the 20^{th} to 120^{th}, 30^{th} to 110^{th}, 30^{th} to 50^{th}, or 90^{th} to 110^{th} amino acid region.

According to one embodiment of the present invention, the improved activity of the glyceraldehyde 3-phosphate dehydrogenase may be achieved by substitution of one or more of the amino acids at positions 36, 37 and 100 in the amino acid sequence of the gene encoding the glyceraldehyde 3-phosphate dehydrogenase.

In one example of the present invention, a *Corynebacterium glutamicum* mutant strain having a new amino acid sequence of the gapA gene was obtained by leucine (Leu)-to-serine (Ser) substitution at position 36 and threonine (Thr)-to-lysine (Lys) substitution at position 37 in the amino acid sequence of the gapA gene encoding glyceraldehyde 3-phosphate dehydrogenase of the *Corynebacterium glutamicum* strain. This *Corynebacterium glutamicum* mutant strain may comprise the gapA gene represented by the amino acid sequence of SEQ ID NO: 3.

In another example of the present invention, a *Corynebacterium glutamicum* mutant strain having a new amino acid sequence of the gapA gene was obtained by leucine (Leu)-to-serine (Ser) substitution at position 36, threonine (Thr)-to-lysine (Lys) substitution at position 37, and phenylalanine (Phe)-to-valine (Val) substitution at position 100 in the amino acid sequence of the gapA gene encoding glyceraldehyde 3-phosphate dehydrogenase of the *Corynebacterium glutamicum* strain. This *Corynebacterium glutamicum* mutant strain may comprise the gapA gene represented by the amino acid sequence of SEQ ID NO: 5.

This *Corynebacterium glutamicum* mutant strain having mutations in the glyceraldehyde 3-phosphate dehydrogenase gene or the amino acid sequence encoding the same may have enhanced L-lysine productivity.

"Enhanced productivity" as used in the present invention means that L-lysine productivity of the mutant strain is higher than that of the parent strain. The parent strain refers to a wild-type strain to be mutated or a mutant strain, and includes a strain that is to be mutated directly or to be transformed with a recombinant vector or the like. In the present invention, the parent strain may be a wild-type *Corynebacterium glutamicum* strain or a strain mutated from the wild-type strain.

According to one embodiment of the present invention, the parent strain may be a *Corynebacterium glutamicum* strain having weakened activity of citrate synthase involved in the lysine biosynthesis pathway (Korean Patent Application No. 10-2021-0050318) (hereinafter referred to as *"Corynebacterium glutamicum* DS2 strain").

In one example of the present invention, as the *Corynebacterium glutamicum* mutant strain having enhanced L-lysine productivity contains mutations in the amino acid sequence of the gapA gene encoding glyceraldehyde 3-phosphate dehydrogenase, it may exhibit increased L-lysine productivity compared to the parent strain due to an increase in the supply of NADPH required for L-lysine biosynthesis. In particular, the *Corynebacterium glutamicum* mutant strain may show an increase in L-lysine production of 50 or more, specifically 5 to 20%, compared to the parent strain, and thus produce 70 g or more, specifically 70 to 85 g of L-lysine, per liter of the strain culture medium.

The *Corynebacterium glutamicum* mutant strain according to one embodiment of the present invention may be obtained through a recombinant vector containing a variant resulting from substitution of a portion of the amino acid sequence of the gapA gene encoding glyceraldehyde 3-phosphate dehydrogenase in the parent strain.

"Portion" as used in the present invention means not all of the amino acid sequence, nucleotide sequence, or polynucleotide sequence, and may be, but is not limited to, 1 to 300, preferably 1 to 100, more preferably 1 to 50.

"Variant" as used in the present invention refers to a variant resulting from substitution of one or more amino acids in the 10^{th} to 130^{th} amino acid region in the amino acid sequence of the glyceraldehyde 3-phosphate dehydrogenase gene involved in the biosynthesis of L-lysine.

According to one embodiment of the present invention, the variant resulting from substitution of the amino acids at positions 36 and 37 in the amino acid sequence of the glyceraldehyde 3-phosphate dehydrogenase gene may have the amino acid sequence of SEQ ID NO: 3 or the nucleotide sequence of SEQ ID NO: 4.

According to one embodiment of the present invention, the variant resulting from substitution of the amino acids at positions 36, 37 and 100 in the amino acid sequence of the glyceraldehyde 3-phosphate dehydrogenase gene may have the amino acid sequence of SEQ ID NO: 5 or the nucleotide sequence of SEQ ID NO: 6.

"Vector" as used in the present invention refers to an expression vector capable of expressing a protein of interest in a suitable host cell, and means a gene construct that contains essential regulatory elements operably linked so that an inserted gene is expressed. Here, "operably linked" means that a gene to be expressed and the regulatory sequence thereof are functionally linked to each other in a manner enabling gene expression. "Regulatory elements" includes a promoter for initiating transcription, any operator sequence for controlling transcription, a sequence encoding suitable mRNA ribosome binding sites, and a sequence for controlling termination of transcription and translation. Examples of this vector include, but are not limited to, plasmid vectors, cosmid vectors, bacteriophage vectors, and viral vectors.

"Recombinant vector" as used in the present invention refers to a recombinant vector that may be transformed into a suitable host cell, and then may replicate regardless of the genome of the host cell or may be integrated into the genome itself. In this case, the "suitable host cell" may contain a replication origin, which is a particular nucleotide sequence which enables the vector to replicate in the suitable host cell and from which replication starts.

The transformation may be performed using a suitable vector introduction technique selected depending on the host cell, so that the gene of interest may be expressed in the host cell. For example, introduction of the vector may be performed by electroporation, heat-shock, calcium phosphate (CaPO₄) precipitation, calcium chloride (CaCl₂) precipitation, microinjection, polyethylene glycol (PEG) method, DEAE-dextran method, cationic liposome method, lithium acetate-DMSO method, or a combination thereof. For the transformed gene, there is no limitation on whether the gene is inserted into the chromosome of the host cell or located outside of the chromosome, as long as the gene may be expressed in the host cell.

The host cell includes a cell transfected, transformed, or infected with the recombinant vector or polynucleotide of the present invention *in vivo* or *in vitro.* The host cell containing the recombinant vector of the present invention may be a recombinant host cell, a recombinant cell, or a recombinant microorganism.

In addition, the recombinant vector according to the present invention may contain a selection marker. The selection marker may be used to select a transformant (host cell) obtained by transformation with the vector. Since only cells expressing the selection marker may survive in the medium treated with the selection marker, the selection marker may select the transformed cells. Representative examples of the selection marker include, but are not limited to, kanamycin, streptomycin, and chloramphenicol.

Genes inserted into the recombinant vector for transformation according to the present invention may be substituted into a host cell such as a *Corynebacterium* sp. microorganism by homologous recombination crossover.

According to one embodiment of the present invention, the host cell may be a *Corynebacterium* sp. strain, for example, a *Corynebacterium glutamicum* DS2 strain.

Another aspect of the present invention provides a method for producing L-lysine, comprising steps of: a) culturing the *Corynebacterium glutamicum* mutant strain in a medium; and b) recovering L-lysine from the mutant strain or the medium in which the mutant strain has been cultured.

The culturing may be performed using a suitable medium and culture conditions known in the art, and any person skilled in the art may easily adjust and use the medium and the culture conditions. Specifically, the medium may be a liquid medium, without being limited thereto. The culturing method include may be, for example, batch culture, continuous culture, fed-batch culture, or a combination thereof, without being limited thereto.

According to one embodiment of the present invention, the medium should meet the requirements of a specific strain in a proper manner, and may be appropriately modified by a person skilled in the art. For the culture medium for a *Corynebacterium* sp. strain, reference may be made to a known document (Manual of Methods for General Bacteriology, American Society for Bacteriology, Washington D.C., USA, 1981), without being limited thereto.

According to one embodiment of the present invention, the medium may contain various carbon sources, nitrogen sources, and trace element components. Examples of the carbon sources that may be used include: sugars and carbohydrates such as glucose, sucrose, lactose, fructose, maltose, starch, and cellulose; oils and fats such as soybean oil, sunflower oil, castor oil, and coconut oil; fatty acids such as palmitic acid, stearic acid, and linoleic acid; alcohols such as glycerol and ethanol; and organic acids such as acetic acid. These substances may be used individually or as a mixture, without being limited thereto. Examples of the nitrogen sources that may be used include peptone, yeast extract, meat extract, malt extract, corn steep liquor, soybean meal, and urea, or inorganic compounds such as ammonium sulfate, ammonium chloride, ammonium phosphate, ammonium carbonate, and ammonium nitrate. The nitrogen sources may also be used individually or as a mixture, without being limited thereto. Examples of phosphorus sources that may be used include, but are not limited to, potassium dihydrogen phosphate or dipotassium hydrogen phosphate or the corresponding sodium-containing salts. In addition, the culture medium may contain, but is not limited to, metal salts such as magnesium sulfate or iron sulfate, which are required for growth. In addition, the culture medium may contain essential growth substances such as amino acids and vitamins. Moreover, suitable precursors may be added to the culture medium. The medium or individual components may be added to the culture medium batchwise or in a continuous manner by a suitable method during culturing, without being limited thereto.

According to one embodiment of the present invention, the pH of the culture medium may be adjusted by adding compounds such as ammonium hydroxide, potassium hydroxide, ammonia, phosphoric acid and sulfuric acid to the microorganism culture medium in an appropriate manner during the culturing. In addition, during the culturing, foaming may be suppressed using an anti-foaming agent such as a fatty acid polyglycol ester. Additionally, to keep the culture medium in an aerobic condition, oxygen or an oxygen-containing gas (for example, air) may be injected into the culture medium. The temperature of the culture medium may be generally 20°C to 45°C, for example, 25°C to 40°C. The culturing may be continued until a desired amount of a useful substance is produced. For example, the culturing time may be 10 hours to 160 hours.

According to one embodiment of the present invention, in the step of recovering L-lysine from the cultured mutant strain or the medium in which the mutant strain has been cultured, the produced L-lysine may be collected or recovered from the medium using a suitable method known in the art depending on the culture method. Examples of the method include, but are not limited to, centrifugation, filtration, extraction, spraying, drying, evaporation, precipitation, crystallization, electrophoresis, fractional dissolution (e.g., ammonium sulfate precipitation), and chromatography (e.g., ion exchange, affinity, hydrophobicity and size exclusion).

According to one embodiment of the present invention, the step of recovering L-lysine may be performed by centrifuging the culture medium at a low speed to remove biomass and separating the obtained supernatant through ion-exchange chromatography.

According to one embodiment of the present invention, the step of recovering L-lysine may include a process of purifying L-lysine.

### Advantageous Effects

The *Corynebacterium glutamicum* mutant strain according to the present invention is able to produce L-lysine in an improved yield as a result of improving the activity of glyceraldehyde 3-phosphate dehydrogenase by mutagenesis of amino acids in the gene encoding the glyceraldehyde 3-phosphate dehydrogenase.

### Brief Description of Drawings

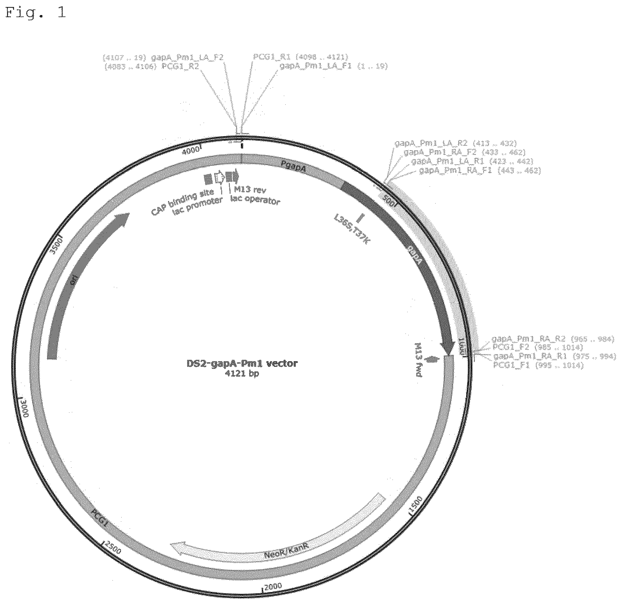

FIG. 1 shows the structure of a DS2-gapA-Pm1 vector containing a gapA gene obtained by leucine-to-serine substitution at amino acid position 36 and threonine-to-lysine substitution at amino acid position 37 in the amino acid sequence of the gapA gene according to one example of the present invention.

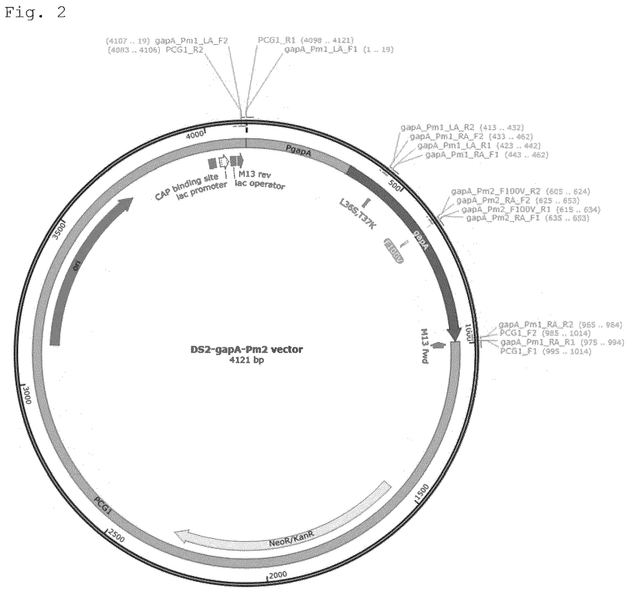

FIG. 2 shows the structure of a DS2-gapA-Pm2 vector containing a gapA gene obtained by leucine-to-serine substitution at amino acid position 36, threonine-to-lysine substitution at amino acid position 37, and phenylalanine-to-valine substitution at amino acid position 100 in the amino acid sequence of the gapA gene according to one example of the present invention.

### Mode for Invention

Hereinafter, the present invention will be described in more detail. However, this description is provided by way of example only to aid the understanding of the present invention, and the scope of the present invention is not limited by this illustrative description.

### Example 1. Construction of Corynebacterium glutamicum Mutant Strain

To construct a *Corynebacterium glutamicum* mutant strain having improved activity of glyceraldehyde 3-phosphate dehydrogenase, a *Corynebacterium glutamicum* DS2 strain as a parent strain and *E. coli* DH5a (HIT Competent cells^{™}, Cat No. RH618) were used.

The *Corynebacterium glutamicum* DS2 strain was cultured in a CM-broth medium (pH 6.8) containing, per liter of distilled water, 5 g of glucose, 2.5 g of NaCl, 5.0 g of yeast extract, 1.0 g of urea, 10.0 g of polypeptone and 5.0 g of beef extract at a temperature of 30°C.

The *E. coli* DH5a was cultured in an LB medium containing, per liter of distilled water, 10.0 g of tryptone, 10.0 g of NaCl and 5.0 g of yeast extract at a temperature of 37°C.

The antibiotics kanamycin and streptomycin used were purchased from Sigma.

DNA sequencing was performed by Macrogen.

### 1-1. Construction of Recombinant Vector

In order to increase L-lysine productivity by increasing the supply of NADPH required for the lysine biosynthesis pathway, the activity of glyceraldehyde 3-phosphate dehydrogenase was improved to produce NADPH instead of NADH. In the method used in this Example, specific mutations in the gapA gene encoding glyceraldehyde 3-phosphate dehydrogenase were induced. Leucine-to-serine substitution at amino acid position 36, and threonine-to-lysine substitution at amino acid position 37 in the amino acid sequence of the gapA gene were performed, and a 442-bp region of the left arm and a 552-bp region of the right arm with respect to the region including 36^{th} and 37^{th} amino acids of the gapA gene on the *Corynebacterium glutamicum* genome were amplified by PCR, ligated by overlap PCR, and then cloned into the recombinant vector pCGI (see Kim et al., Journal of Microbiological Methods 84 (2011) 128-130). The resulting plasmid was named DS2-gapA-Pm1 (see FIG. 1) . For construction of the plasmid, the primers shown in Table 1 below were used to amplify each gene fragment.

**[Table 1]**

| Primer name and sequence (5'-3') | | | SEQ ID NO. |
|---|---|---|---|
| Primers for amplification of left homology arm of gapA | gapA_Pm1_LA_F1 | agtgcgaacgatttcaggt | 7 |
| | gapA_Pm1_LA_F2 | gaccatgattacgccagtgcgaacgatttca ggt | 8 |
| | gapA_Pm1_LA_R1 | ggagtcgttgactgcaacta | 9 |
| | gapA_Pm1_LA_R2 | actgcaactacctcgagatc | 10 |
| Primers for amplification of right homology arm of gapA | gapA_Pm1_RA_F1 | aaggacaacaagaccctttc | 11 |
| | gapA_Pm1_RA_F2 | caacgactccaaggacaacaagaccctttc | 12 |
| | gapA_Pm1_RA_R1 | aaccagagcaacagccttag | 13 |
| | gapA_Pm1_RA_R2 | acagccttagctgcaccggt | 14 |

PCR was performed using the above primers under the following conditions. Using a thermocycler (TP600, TAKARA BIO Inc., Japan), and a reaction solution containing 100 µM of each deoxynucleotide triphosphate (dATP, dCTP, dGTP, dTTP), 1 pM of oligonucleotide, and 10 ng of the chromosomal DNA of *Corynebacterium glutamicum* ATCC 13032 as a template, PCR was performed for 25 to 30 cycles in the presence of 1 unit of a pfu-X DNA polymerase mixture (Solgent). The PCR cycles each consisted of (i) denaturation at 94°C for 30 sec, (ii) annealing at 58°C for 30 sec, and (iii) extension at 72°C for 1 to 2 min (a polymerization time of 2 min per kb).

The gene fragments produced as described above were cloned into the pCGI vector by self-assembly cloning. The vector was transformed into *E. coli* DH5a, which was then streaked on an LB-agar plate containing 50 ug/ml of kanamycin, and cultured at 37°C for 24 hours. After the finally formed colonies were isolated and it was checked whether the inserts would be exactly present in the vector, the vector was isolated and used for recombination of the *Corynebacterium glutamicum* strain.

As the process commonly performed in the above method, the genes of interest were amplified from the genomic DNA of *Corynebacterium glutamicum* ATCC 13032 by PCR and inserted into the pCGI vector by self-assembly cloning according to the strategy, followed by selection in *E*. *coli* DH5a. For chromosomal base substitution, the gene fragments were amplified individually and ligated by overlap PCR to obtain a target DNA fragment. During genetic manipulation, Ex Taq polymerase (Takara) and Pfu polymerase (Solgent) were used as PCR amplification enzymes, and various restriction enzymes and DNA modifying enzymes used were purchased from NEB. These polymerases and enzymes were used according to the supplied buffer and protocols.

### 1-2. Construction of Mutant Strain

A DS2-1 strain, a mutant strain, was constructed using the DS2-gapA-Pm1 vector. The vector was prepared at a final concentration of 1 µg/µl or higher, and introduced into the *Corynebacterium glutamicum* DS2 strain by electroporation (see Tauch et al., FEMS Microbiology Letters 123 (1994), 343-347), thus inducing primary recombination. At this time, the electroporated strain was plated on a CM-agar plate containing 20 µg/µl of kanamycin, and the colonies were isolated, and then whether the vector would be properly inserted into the induced position on the genome was analyzed by PCR and sequencing. In order to induce secondary recombination, the isolated strain was inoculated into a CM-agar liquid medium containing streptomycin, cultured overnight or longer, and then plated on an agar medium containing streptomycin at the same concentration, and the colonies were isolated. After it was checked whether the final isolated colonies would have resistance to kanamycin, whether mutation was introduced into the gapA gene in the strains having no antibiotic resistance was analyzed by sequencing (see Schafer et al., Gene 145 (1994) 69-73). Finally, a *Corynebacterium glutamicum* mutant strain (DS2-1) having the mutant gapA gene introduced thereinto was obtained.

### Example 2. Construction of Corynebacterium glutamicum Mutant Strain

In order to increase L-lysine productivity by increasing the supply of NADPH required for the lysine biosynthesis pathway, the activity of glyceraldehyde 3-phosphate dehydrogenase was improved to produce NADPH instead of NADH. In the method used in this Example, specific mutations in the gapA gene encoding glyceraldehyde 3-phosphate dehydrogenase were induced. Leucine-to-serine substitution at amino acid position 36, threonine-to-lysine substitution at amino acid position 37, and phenylalanine-to-valine at amino acid position 100 in the amino acid sequence of the gapA gene were performed, and a 442-bp region of the left arm and a 360-bp region of the right arm with respect to the region including 36^{th}, 37^{th} and 100^{th} amino acids of the gapA gene on the *Corynebacterium glutamicum* genome were amplified by PCR, ligated by overlap PCR, and then cloned into the recombinant vector pCGI (see Kim et al., Journal of Microbiological Methods 84 (2011) 128-130). The resulting plasmid was named DS2-gapA-Pm2 (see FIG. 2). For construction of the plasmid, the primers shown in Table 2 below were used to amplify each gene fragment.

**[Table 2]**

| Primer name and sequence (5'-3') | | | SEQ ID NO. |
|---|---|---|---|
| Primers for amplification of left homology arm of gapA | gapA_Pm1_LA_F1 | agtgcgaacgatttcaggt | 7 |
| | gapA_Pm1_LA_F2 | gaccatgattacgccagtgcgaacgatttcaggt | 8 |
| | gapA_Pm1_LA_R1 | ggagtcgttgactgcaacta | 9 |
| | gapA_Pm1_LA_R2 | actgcaactacctcgagatc | 10 |
| Primers for amplification of right homology arm of gapA | gapA_Pm2_RA_F 1 | ttcaccgatgcaaacgcgg | 15 |
| | gapA_Pm2_RA_F2 | caccggcgtcttcaccgatgcaaacgcgg | 16 |
| | gapA_Pm1_RA_R1 | aaccagagcaacagccttag | 13 |
| | gapA_Pm1_RA_R2 | acagccttagctgcaccggt | 14 |

Thereafter, the same method as Example 1 was performed using the DS2-gapA-Pm2 vector, thus constructing and obtaining a mutant strain, DS2-2 strain.

### Experimental Example 1. Comparison of L-Lysine Productivity Between Parent Strains and Mutant Strain

L-lysine productivity was compared between the parent strain *Corynebacterium glutamicum* DS2 strain and the L-lysine-producing mutant strains DS2-1 and DS2-2 strains constructed in Examples 1 and 2.

Each of the strains was inoculated into a 100-ml flask containing 10 ml of a lysine medium having the composition shown in Table 3 below, and then cultured with shaking at 180 rpm at 30°C for 28 hours. After completion of the culture, the amount of L-lysine produced was measured by HPLC (Shimazu, Japan), and the results of the measurement are shown in Table 4 below.

**[Table 3]**

| Composition | Content (per L of distilled water) |
|---|---|
| Glucose | 100 g |
| Ammonium sulfate | 55 g |
| KH₂PO₄ | 1.1 g |
| MgSO₄ □ H₂O | 1.2 g |
| MnSO₄ □ H₂O | 180 mg |
| FeSO₄ □ H₂O | 180 mg |
| Thiamine □ HCl | 9 mg |
| Biotin | 1.8 mg |
| CaCO₃ | 5% |
| pH | 7.0 |

**[Table 4]**

| Strain | OD₆₁₀ | L-lysine (g/L) | L-lysine production per gram dry cell weight (g/gDCW) |
|---|---|---|---|
| Parent strain (DS2) | 23.0 | 69.7 | 7.2 |
| Mutant strain (DS2-1) | 22.5 | 76.3 | 8.1 |
| Mutant strain (DS2-2) | 22.4 | 75.2 | 8.0 |

As shown in Table 4 above, it was confirmed that, in the *Corynebacterium glutamicum* mutant strains DS2-1 and DS2-2 in which specific positions (amino acids at positions 36 and 37, or amino acids at positions 36, 37 and 100) in the amino acid sequence of the gapA gene were substituted with optimal amino acid residues to enhance the lysine biosynthesis pathway, the L-lysine productivities of the mutant strains increased by about 12.5% and 11.1%, respectively, compared to that of the parent strain *Corynebacterium glutamicum* DS2 strain. From these results, it could be seen that the mutations in the gapA gene mutation enhanced the L-lysine productivity of the strain by improving the activity of glyceraldehyde 3-phosphate dehydrogenase.

So far, the present invention has been described with reference to the embodiments thereof. Those of ordinary skill in the art to which the present invention pertains will appreciate that the present invention may be embodied in modified forms without departing from the essential characteristics of the present invention. Therefore, the disclosed embodiments should be considered from an illustrative point of view, not from a restrictive point of view. The scope of the present invention is defined by the claims rather than the foregoing description, and all differences within the scope equivalent thereto should be construed as being included in the present invention.

## Claims

1. A *Corynebacterium glutamicum* mutant strain having enhanced L-lysine productivity by having improved activity of glyceraldehyde 3-phosphate dehydrogenase.

2. The *Corynebacterium glutamicum* mutant strain of claim 1, wherein the improved activity of the glyceraldehyde 3-phosphate dehydrogenase is achieved by site-directed mutagenesis of a gene encoding the glyceraldehyde 3-phosphate dehydrogenase.

3. The *Corynebacterium glutamicum* mutant strain of claim 1, wherein the improved activity of the glyceraldehyde 3-phosphate dehydrogenase is achieved by substitution of one or more of amino acids at positions 36, 37 and 100 in an amino acid sequence of a gene encoding the glyceraldehyde 3-phosphate dehydrogenase.

4. The *Corynebacterium glutamicum* mutant strain of claim 1, wherein the mutant strain comprises the amino acid sequence represented by SEQ ID NO: 3 or 5.

5. A method for producing L-lysine, comprising steps of:
a) culturing the *Corynebacterium glutamicum* mutant strain of any one of claims 1 to 4 in a medium; and
b) recovering L-lysine from the mutant strain or the medium in which the mutant strain has been cultured.
